# EUROPEAN PATENT SPECIFICATION

(11) **EP 1 572 276 B1**
(45) Date of publication and mention of the grant of the patent: **09.05.2007**
(21) Application number: 02783396.1
(22) Date of filing: 20.11.2002
(51) Int. Cl.: A61M 16/08, A61M 16/10, B01D 53/26

(54) **DEVICE AND METHOD FOR THE GENERATION OF RESPIRATIONAL AIR**
VORRICHTUNG UND VERFAHREN ZUR ERZEUGUNG VON BEATMUNGSLUFT
DISPOSITIF ET PROCEDE DE PRODUCTION D'AIR RESPIRATOIRE

(43) Date of publication of application: 14.09.2005
(73) Proprietor: Imt Medical AG, 9470 Buchs SG (CH)
(72) Inventor: FRIBERG, Harri, FL-9493 Mauren (CH); DÄSCHER, Jakob, CH-7306 Fläsch (CH)
(74) Representative: Rosenich, Paul
(86) International application number: PCT/IB2002/004838
(87) International publication number: WO 2004/045695

(56) References cited:
- US-A- 4 080 103

## Description

Die Erfindung betrifft eine Vorrichtung und ein Verfahren zur Erzeugung der Beatmungsluft.

Es gab bisher mehrere Bestrebungen, trockene Beatmungsluft zu erzeugen. Es ist bekannt, dass wenn Gas bzw. Luft in einem Raum komprimiert wird, ihr Feuchtigkeitsaufnahmevermögen gross ist und beim Abkühlen der Luft z.B. in einem Speicherbehälter ein Teil der aufgenommenen Feuchtigkeit kondensieren kann, was unerwünscht ist. Auch ist für die Beatmung selbst nur eine gewisse Luftfeuchtigkeit erwünscht. Beatmungsluft aus einem Kompressor muss daher sowohl aus technischen wie auch aus gesundheitlichen Gründen entfeuchtet werden.

### (Definitionen)

- Unter Beatmungsluft im Sinne der Erfindung ist jene Gas oder Luftmischung zu verstehen, die einem Patienten maschinell zugeführt werden kann.
- Unter Feuchtigkeit im Sinne der Erfindung ist Wasser oder eine andere Flüssigkeit oder Flüssigkeitsmischung im flüssigen oder gas- oder luftförmigen Aggregatszustand zu verstehen.
- Als Düsenform ist die Form der Ausflussöffnung zu verstehen, die verschiedene Ausführungsmöglichkeiten aufweisen kann.
- Unter Durchgangsverjüngung im Sinne der Erfindung ist aufgrund eines verjüngten Durchflusses insbesondere eine Blende oder eine Düse in einem Rohr zu verstehen, in der wegen des einseitig höheren Drucks einerseits eine lokale Erhöhung der Strömungsgeschwindigkeit, andererseits eine lokale, tiefere Temperatur der Beatmungsluft nach der DurchgangsverJüngung Im Verhältnis zur Temperatur vor der Durchgangsverjüngung produziert wird.

Zum Stand der Technik gehören Vorrichtungen, welche das obengenannte Entfeuchtungsproblem durch mechanische Abscheider mit Mikrofilter, durch Wärmebehandlung der Luft oder mit der Hilfe von chemischen Trocknungssystemen (z.B. mit Salz) teilweise gelöst haben.

Die US 4080103 offenbart ein portables Luftkompressorsystem für ein Beatmungsgerät, welches durch eine Kraftquelle betrieben wird. Das System ist mit einer Mehrzahl von Kompressoren ausgestattet Jeder Kompressor hat einen Ventilator um Luft durch den Kompressor strömen zu lassen und hat einen Ausgang für komprimierte Luft. Das System ist mit einer Kühlschlange mit einem Eingang und einem Ausgang ausgestattet welche so positioniert ist, dass die Ventilatoren Luft durch die Luftschlange treiben. Leitungen verbinden die Ausgänge des Kompressors mit dem Eingang der Kühlschlange. Das System ist mit einem Dampftrockner ausgestattet. Leitungen verbinden den Ausgang der Kühlschlange mit dem Dampftrockner. Der Eingang eines Luftbehälters ist verbunden mit dem Dampftrockner und dessen Ausgang kann mit dem Beatmungsgerät verbunden werden. Ein Druckregler steht Im Kontakt mit dem Luftbehälter. Ein Druckschalter ist mit dem Regler und mit mindestens einem Kompressor zu dessen Unterbrechung verbunden, für den Fall, dass Im Luftbehälter ein vorgegebener Druck erreicht ist.

Der Erfinder erkannte, dass die bekannten Systeme nachteilig sind in Bezug auf die folgenden Punkte:
a) Die bisherigen Vorrichtungen erzielten eine aufwendige oder ungenügende Entfeuchtung von Beatmungsluft. Bei vielen kann die Abscheidung vom Wasser aus dem Gas bzw. der Luft nur teilweise verwirklicht werden. Das bedeutet, dass eine optimale Luftfeuchtigkeit durch die bekannten Methoden bisher nur mit grossem Aufwand oder gar nicht zustandegebracht wurde.

Der Erfindung liegt somit die Aufgabe zugrunde, eine Vorrichtung und ein Verfahren zu entwickeln, bei denen die Beatmungsluft mit guter Qualität erzeugt wird und durch die der Feuchtigkeitsgehalt ein Optimum erreichen kann, ohne den Aufwand zu weit zu treiben.

Gelöst wird diese Aufgabe durch
i) eine Durchgangsverjüngung in einem Rohr der Vorrichtung mit einer Düse und anschliessender Wasserabscheidung mittels herkömmlichem Wasserabscheiders. Die Düse kann auch nach dem Wasserabscttneider montiert werden.
ii) den physikalischen Effekt, nach dem im Bereich der Düse die Mischung von Gas bzw. Luft und Wasser wegen des Temperaturabfalls nach der Düse - der infolge des Druckabfalls nach der Verjüngung entsteht - in einen physikalischen Zustand kommt, in dem das Wasser aus dem Gas bzw. aus der Luft besser auskondensiert bzw. abgeschieden werden kann.
iii) den Effekt, bei dem das auskondensierte Wasser durch den Luftstrom mitgerissen und unmittelbar nach der Düse in einem Wasserabscheider abgeschieden werden kann, noch bevor sich die Beatmungsluft mit dem auskondensierten Wasser wieder sättigen kann.
iiii) Bevor die Mischung von Gas bzw. Luft und Wasser in der Düse ankommt, wird sie zuerst in einem Radiator von Ventilatoren abgekühlt, um auf eine niedrigere Temperatur zu kommen.

Durch den vorgängig beschriebenen Entfeuchtungsprozess von Beatmungsluft werden in den folgenden Schritten die nachstehenden Verbesserungen erreicht:
- Wegen der Durchgangsverjüngung (mit einer Düse) im Rohr ergibt sich vor der Düse ein höherer Druck und eine Erhöhung von der Strömungsgeschwindigkeit in der Verjüngung sowie danach ein Druckabfall durch den entsprechend die Temperatur der Gas oder Luftmischung fällt, wodurch eine optimale Wasserabscheidung ermöglicht wird.
- Der von dem Abscheidevorgang benötigte Druck wird z.B. bei einem Versuchsaufbau von Manometern und die Temperatur von Temperatursensoren im Rohr gemessen.

Die Lösung des grundsätzlichen Erfindungsgedankens kann in verschiedenen Ausführungsformen realisiert werden.

Die Düsenverjüngung, welche die bestimmendste Rolle bei der erfindungsgemässen Vorrichtung spielt, kann verschiedene Formen aufweisen, die einen Einfluss auf die Geschwindigkeit des Durchflusses ausüben können.

Falls die Düse eine scharfkantige Form hat, ist der Geschwindigkeitsbeiwert beim Durchfluss hoch (0,97 ϕ).Bei dieser Ausführungsmöglichkeit sind die Breiten von Eingang und Ausgang gleich.

Nach einer anderen Variante ist die Düsenform abgerundet, der Durchgang ist ziemlich lang und die Breite vom Eingang ist etwa doppelt so gross wie die vom Ausgang, dementsprechend eine noch höhere Durchflussgeschwindigkeit (0,97-0,99 ϕ) erzeugt wird.

Als Alternative kann die Düse über eine zylinder oder eckige Form verfügen, bei der der Durchgang im Vergleich mit den vorherigen Lösungen am längsten ist und der Eingang eine grössere Breite aufweist, als der Ausgang. Bei dieser Variante erreicht die Geschwindigkeit beim Durchfluss einen niedrigeren Wert (0,82 ϕ).

Die Bezugszeichenliste und die Zeichnung sind zusammen mit den in den Ansprüchen beschriebenen, beziehungsweise geschützten Gegenständen integrierender Bestandteil der Offenbarung dieser Anmeldung.

### Figurenbeschreibung

Die Figuren werden zusammenhängend und übergreifend beschrieben. Gleiche Bezugszeichen bedeuten gleiche Bauteile, Bezugszeichen mit unterschiedlichen Indices geben funktionengleiche Bauteile an.

Es zeigen dabei:
- Fig.1: die erfindungsgemässe Vorrichtung zur Erzeugung von Beatmungsluft als Blockschaltbild dar;
- Fig.2: eine ausführliche Darstellung des Entfeuchtungsprozesses mit der erfindungsgemässen Vorrichtung;
- Fig.3: die verschiedenen Ausführungsformen von der Düse dar;

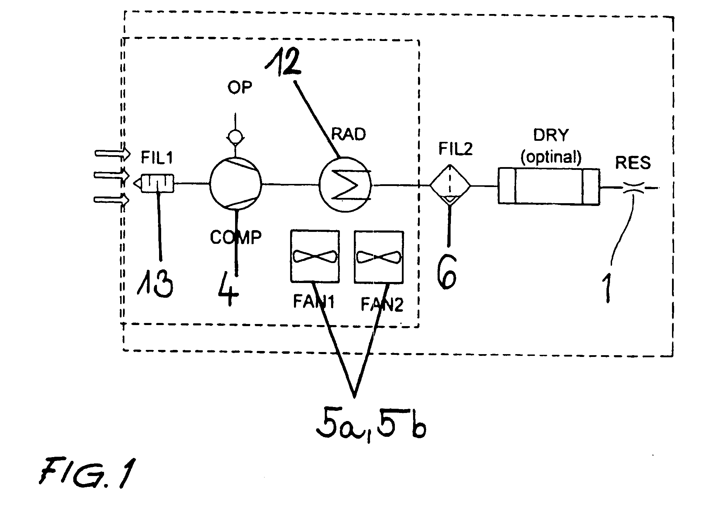

Fig.1 stellt ein Schaltdiagramm (Blockdiagramm) eines erfindungsgemässen Kompressors 4 dar.
Auf der linken Seite befindet sich ein Luftansaugeinlass mit einem Schalldämpfer 13 (FIL1). Im Anschluss daran ist der Kompressor 4 dargestellt mit einem Überdruckventil 5, an dessen Ausgang ein Kühlerradiator 12 angeschlossen ist, der durch Ventilatoren 5a, 5b (FAN1, FAN 2) mit Kühlluft beblasbar ist und damit die Temperatur der Druckluft sinken kann.

Im Anschluss an den Radiator 12 befindet sich ein Mikrofilter mit Wasserabscheider 6 (FIL2), der die Aufgabe hat, überschüssiges Wasser, das aus der komprimierten Luft auskondensiert ist, aus der Luftleitung zu entfernen. Das mit "RES" bezeichnete Bauteil 1 beinhaltet die Erfindung, nämlich die Durchgangsverjüngung die Feuchtigkeit aus dem Raum nach der erfindungsgemässen Düse entfernt.

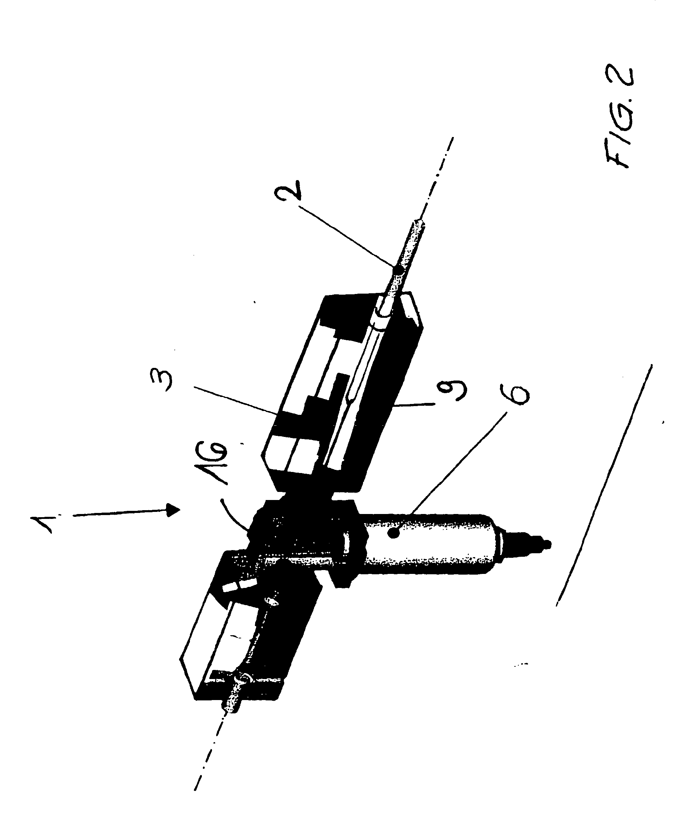

Fig. 2 stellt den Entfeuchtungsprozess mit der erfindungsgemässen Vorrichtung 1 dar. Bevor die im Rohr 2 fliessende Mischung von entweder Gas oder Luft und Wasser die Durchgangsverjüngung 9 - die eine Düsenform 3 aufweisen kann - erreichen würde, wird sie bevorzugt zuerst von Ventilatoren 5a, 5b im Radiator 12 abgekühlt (Fig. 1). Durch die Verjüngung 9 in der Düse 3 bietet sich vor ihr ein höherer Druck und eine lokale Erhöhung der Strömungsgeschwindigkeit sowie danach ein Druckabfall, dem zufolge ein weiterer, wesentlich grösserer Temperaturabfall in der Gas- oder Luftmischung. Das Wasser kondensiert durch die lokale, tiefe Temperatur in der Düse 3 aus dem Gas aus. Das auskondensierte Wasser wird durch den Luftstrom mitgerissen und kann unmittelbar nach der Düse 3 in einem weiteren Wasserabscheider 6 mit einem Filter 16 abgeschieden werden, bevor sich entweder das Gas oder die Luft aufgrund der Umgebungstemperatur wieder sättigt.

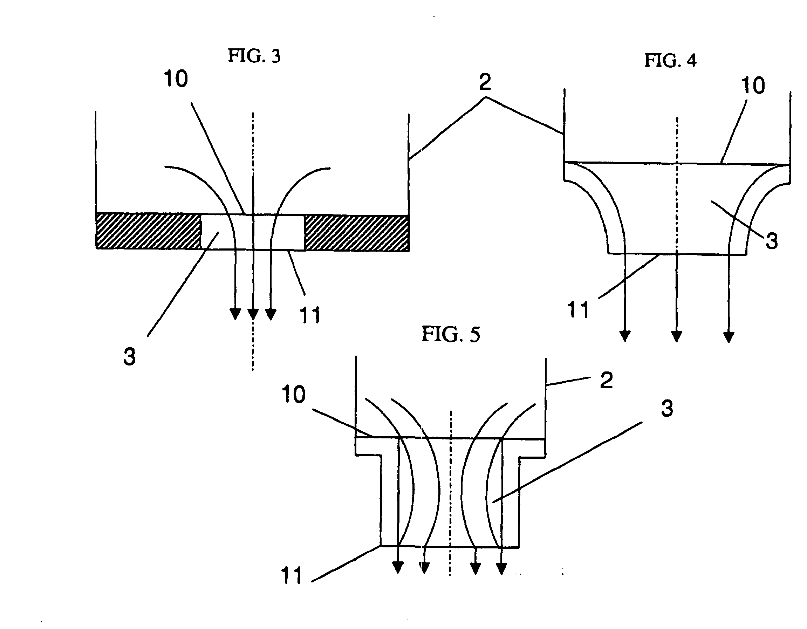

Fig. 3-5 zeigen eine Darstellung von Ausführungsformen der Düse 3. Die Düse 3, die als Verjüngung 9 vom Rohr 2 beinhaltet ist, kann - wie schon weiter oben ausgeführt - verschiedene Formen aufweisen, von denen die Durchflussgeschwindigkeit und damit die Gas- oder Luftmischung abhängig ist.

Bei dem Aufbau gemäss Fig. 3 verfügt die Düse 3 über eine scharfkantige Form, bei der sowohl der Eingang 10 als auch der Ausgang 11 die gleiche Grösse haben. Der Durchgang ist weiterhin dadurch charakterisiert, dass seine Länge und Breite ziemlich kurz sind. Bei dieser Ausführungsmöglichkeit ist die Geschwindigkeit des Durchflusses relativ hoch.

Aus der Fig. 4 ist eine abgerundete Düsenform 3 zu sehen, bei welcher der Eingang 10 doppelt so gross ist als der Ausgang 11. Der Durchgang ist wesentlich länger und breiter, als bei der Fig.3. Wegen der vorher erwähnten Eigenschaften vom Durchgang ist die Strömungsgeschwindigkeit bei dieser Ausführungsform besonders hoch.

Fig. 5 stellt eine zylinderförmige oder eckige Düse 3 dar, bei der sowohl die Breite als auch die Länge vom Durchgang ziemlich hoch sind. Der eckigen, breiten und langen Form infolge ist die Geschwindigkeit des Durchflusses im Verhältnis der vorherigen Lösungen viel langsamer.

### Bezugszeichenliste

| | | |
|---|---|---|
| 1- | Vorrichtung | |
| 2- | Rohr | |
| 3- | Düse | |
| 4- | Kompressor | |
| 5- | Ventilator | 5a, 5b |
| 6- | Wasserabscheider | |
| 7- | Beatmungsluft | |
| 8- | Feuchtigkeitsgehalt | |
| 9- | Durchgangsverjüngung | |
| 10- | Eingang | |
| 11- | Ausgang | |
| 12- | Radiator | |
| 13- | Schalldämpfer | |

## Claims

1. Device (1) for the generation of respirational air (7), comprising a compressor (4), from which compressed gas is delivered in a tube (2), comprising a device for cooling (5) and comprising at least one water separator (6), **characterized in that** the tube (2) contains a tapering passage (9) after which the water separator (6) is directly connected, the pressure drop after the tapering passage (9) having a cooling effect on the gas in the operating state, a nozzle (3) preferably being provided as tapering passage (9).

2. Device (1) according to Claim 1, **characterized in that** the nozzle (3) may have different forms.

3. Device (1) according to either of the preceding claims, **characterized in that** a second water separator (6) is connected before the tapering passage (9).

4. Device (1) according to Claim 3, **characterized in that** a further cooling device (5) for the gas is provided in the device (1) before the tapering passage (9).

5. Method for the generation of respirational air (7) using a device according to any of the preceding claims, comprising a compressor (4) which delivers compressed gas, after which this gas is passed through a tapering passage (9) in which the gas is cooled, water being precipitated and being separated off by means of water separator (6).

6. Method according to Claim 5, **characterized in that** the gas or air mixture is cooled by at least one fan (5) on the way to the tapering passage (9).

7. Method according to either of the preceding Claims 5 and 6, **characterized in that** water which has condensed out is separated off in the water separator (6) before the tapering passage (9).

## Patentansprüche

1. Vorrichtung (1) zur Erzeugung von Beatmungsluft (7) mit einem Kompressor (4), aus dem komprimiertes Gas in einem Rohr (2) geliefert wird, mit einer Vorrichtung zur Abkühlung (5) und mit wenigstens einem Wasserabscheider (6), **dadurch gekennzeichnet, dass** das Rohr (2) eine Durchgangsverjüngung (9) beinhaltet, nach der der Wasserabscheider (6) unmittelbar angeschlossen ist, wobei der Druckabfall nach der Durchgangsverjüngung (9) im Betriebszustand einen Abkühleffekt auf das Gas bewirkt, wobei als Durchgangsverjüngung (9) vorzugsweise eine Düse (3) vorgesehen ist.

2. Vorrichtung (1) nach Anspruch 1, **dadurch gekennzeichnet, dass** die Düse (3) verschiedene Formen aufweisen kann.

3. Vorrichtung (1) nach einem der vorgehenden Ansprüche, **dadurch gekennzeichnet, dass** vor der Durchgangsverjüngung (9) ein zweiter Wasserabscheider (6) angeschlossen ist.

4. Vorrichtung (1) nach Anspruch 3, **dadurch gekennzeichnet, dass** in der Vorrichtung (1) vor der Durchgangsverjüngung (9) eine weitere Kühlvorrichtung (5) für das Gas vorgesehen ist.

5. Verfahren zur Erzeugung von Beatmungsluft (7) mit einer Vorrichtung nach einem der vorhergehenden Ansprüche, mit einem Kompressor (4), der komprimiertes Gas anliefert, wonach dieses Gas durch eine Durchgangsverjüngung (9) geschickt wird, in der das Gas abgekühlt wird, wobei Wasser ausfällt, das mittels Wasserabscheiders (6) ausgeschieden wird.

6. Verfahren nach Anspruch 5, **dadurch gekennzeichnet, dass** die Gas oder Luftmischung am Weg zur Durchgangsverjüngung (9) von wenigsten einem Ventilator (5) abgekühlt wird.

7. Verfahren nach einem der vorhergehenden Ansprüche 5 oder 6, **dadurch gekennzeichnet, dass** schon vor der Durchgangsverjüngung (9) auskondensiertes Wasser im Wasserabscheider (6) abgeschieden wird.

## Revendications

1. Dispositif (1) de production d'air respiratoire (7) comprenant un compresseur (4), duquel un gaz comprimé est fourni dans un tuyau (2), et un dispositif de refroidissement (5) et au moins un séparateur d'eau (6), **caractérisé en ce, que** le tuyau (2) comprend une diminution de passage (9), après quoi le séparateur d'eau (6) est directement relié, la chute de pression après la diminution de passage (9), en état de marche, provoquant un effet de refroidissement au gaz, une buse (3) étant prévue de préférence comme diminution de passage (9).

2. Dispositif (1) selon la revendication 1, **caractérisé en ce, que** la buse (3) peut présenter des formes diverses.

3. Dispositif (1) selon une quelconque des revendications précédentes, **caractérisé en ce**, **qu**'un deuxième séparateur d'eau (6) est relié devant la diminution de passage (9).

4. Dispositif (1) selon la revendication 3, **caractérisé en ce**, **qu**'un autre dispositif de refroidissement (5) pour le gaz est prévu dans le dispositif (1) devant la diminution de passage (9).

5. Procédé de production d'air respiratoire (7) en utilisant un dispositif selon une quelconque des revendications précédentes, comprenant un compresseur (4), qui fournit un gaz comprimé, après quoi ce gaz est envoyé à travers d'une diminution de passage (9), dans laquelle le gaz est refroidi tout en précipitant de l'eau, qui est éliminé au moyens d'un séparateur d'eau (6).

6. Procédé selon la revendication 5, **caractérisé en ce, que** le mélange de gaz ou d'air est refroidi à son chemin vers la diminution de passage (9) par au moins un ventilateur (5).

7. Procédé selon une quelconque des revendications 5 ou 6, **caractérisé en ce, que** l'on sépare de l'eau condensé dans un séparateur d'eau (6) devant la diminution de passage (9).
